(19) Europäisches Patentamt
European Patent Office
Office européen des brevets

(11) Veröffentlichungsnummer: **0 039 027**
**A1**

(12) # EUROPÄISCHE PATENTANMELDUNG


(21) Anmeldenummer: **81102985.9**

(22) Anmeldetag: **18.04.81**

(51) Int. Cl.³: **G 01 N 33/52**
C 12 Q 1/00, G 01 N 31/22

(30) Priorität: **30.04.80 DE 3016618**

(43) Veröffentlichungstag der Anmeldung:
**04.11.81 Patentblatt 81/44**

(84) Benannte Vertragsstaaten:
**AT BE CH DE FR IT LI**



(71) Anmelder: **BEHRINGWERKE AKTIENGESELLSCHAFT**
**Postfach 1140**
**D-3550 Marburg/Lahn(DE)**

(72) Erfinder: **Scharf, Gerhard**
**Wiesenhofstrasse 15**
**D-3560 Biedenkopf-Kombach(DE)**

(74) Vertreter: **Schüler, Albert, Dr. et al,**
**HOECHST AKTIENGESELLSCHAFT Zentrale**
**Patentabteilung Postfach 80 03 20**
**D-6230 Frankfurt am Main 80(DE)**


(54) **Chemische Verbindungen, Verfahren zu ihrer Herstellung, diese enthaltende Mittel sowie ihre Verwendung.**


(57) Es werden an einen Träger gebundene Verbindungen beschrieben, die als Chromogene für in einem Träger enthaltene analytische Indikatoren dienen. Weiterhin wird ein Verfahren zur Herstellung dieser trägergebundenen Chromogene, ein diese enthaltendes Mittel sowie ihre Verwendung zum Nachweis und zur Bestimmung von Bestandteilen von Körperflüssigkeiten und Exkrementen beschrieben. Dazu verwendet man ein Teststäbchen mit verschiedenen Zonen:

- Reaktionszone (2) mit Auffangzone (7);
- Ionenaustauschzone (3);
- Aufbringzone (4);
- Zone zur Regulierung der Fliessgeschwindigkeit (5).


FIG.1

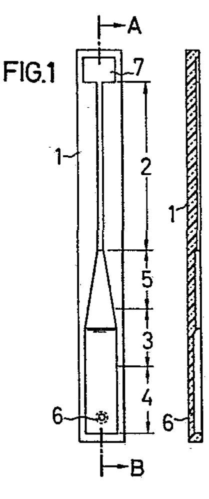


FIG. 2

EP 0 039 027 A1

Chemische Verbindungen, Verfahren zu ihrer Herstellung, diese enthaltende Mittel sowie ihre Verwendung

Die Erfindung betrifft an einen Träger gebundene Verbindungen, die als Chromogene für in einem Träger enthaltene analytische Indikatoren dienen, ein Verfahren zu ihrer Herstellung, diese Verbindungen enthaltende Mittel sowie ihre Verwendung zum Nachweis und zur Bestimmung von Bestandteilen von Körperflüssigkeiten oder Exkrementen.

In solchen Nachweis- und Bestimmungsverfahren werden heutzutage der Einfachheit halber als Teststreifen bekannte Mittel verwendet. Solche Mittel sind beispielsweise in der Deutschen Offenlegungsschrift 29 22 856 beschrieben.

Ein Problem, das beim Gebrauch solcher Teststreifensysteme auftreten kann, besteht darin, daß die in den Testreaktionen gebildeten Indikatoren "ausbluten" können, was zu unscharfer Anzeige und Verlust an Genauigkeit des Testergebnisses führt. Es sind bereits Lösungen vorgeschlagen worden, die jedoch nicht ohne Nachteile sind.

Es wurde nun gefunden, daß vorteilhaft Indikatoren verwendet werden können, die durch, vorzugsweise kovalente, Bindung an ein Trägermaterial an einer Diffusion im Träger gehindert sind, vorzugsweise Chromogene, aus denen im Verlauf der Testreaktion Farbstoffe entstehen.

Bekannt war, in Teströhrchen zur Bestimmung von Ionen als Indikatoren dienende Komplexbildner ionisch an Träger zu binden (Labor Praxis 1980, Heft 3, S. 26 - 29).

Bekannt waren auch Mittel zum Nachweis und zur Bestimmung von Glucose in diese enthaltenden Flüssigkeiten, wobei Glucose katalytisch oxydiert wird und Wasserstoffperoxid entsteht. Zur Quantifizierung der Substratkonzentration oder -menge kann die Bestimmung der Konzentration oder Menge des gebildeten Wasserstoffperoxids dienen. Dazu kann die mittels des Enzyms Peroxidase katalysierte Oxydation von geeigneten Chromogenen verwendet werden. Besonders einfach ist die Verwendung von Teststreifen. Die Testaussage stützt sich hier auf eine Aussage hinsichtlich der gebildeten Farbstoffkonzentration, welche innerhalb eines terminierten Zeitintervalles gebildet worden ist, und kann sowohl subjektiv mittels Vergleich mit einer Farbskala, als auch objektiv mittels Verwendung von Reflektionsphotometern erfolgen. Geeignete Chromogene sind bekannt. Zumindest die subjektive Auswertung kann dabei aber als höchstens halbquantitativ bezeichnet werden.

In Testmitteln zur halbquantitativen oder quantitativen Anzeige kann alternativ die Länge einer gebildeten Farbzone nach Aufbringen einer definierten Menge Probensubstanz als Maß für die Substanzkonzentration oder -menge verwendet werden.

Hierbei stellt sich das Problem, daß die gebildete Indikatorschicht von dem durchströmenden Lösungsmittel teilweise gelöst werden kann und sich, wie bei allen chromatographischen Prozessen bekannt, in Richtung des Laufmittels mitbewegt, weshalb in Teströhrchen zur quantitativen Bestimmung von Ionen (Labor Praxis 1980, Heft 3, S. 26-29, Vogel-Verlag, Würzburg), die als Indikatoren wirksamen Komplexbildner ionisch an Cellulose-Ionenaustauscher als feste Träger gebunden werden.

BAD ORIGINAL

Da die Gesamtladung des Komplexbildners nach der Reaktion mit dem zu untersuchenden Ion nicht verändert wird, verändert sich die Affinität des Indikators zur Cellulose Matrix nicht wesentlich, und die gebildete Indikatorzone wandert kaum beim Durchtritt von überschüssigem Lösungsmittel. Dazu muß allerding die Ionenstärke des Laufmittels klein gehalten werden.

Nun enthält zumindest die Probenflüssigkeit selbst Fremdionen, welche bewirken, daß gewisse Ionenaustauscheffekte mit dem Indikator an der Cellulose Matrix auftreten. Verstärkt wird dieser Effekt, wenn sich größere Mengen Laufmittel hindurchbewegen, da ja zur Einhaltung der gewünschten Reaktionsbedingungen häufig ein bestimmter pH-Bereich eingehalten werden muß, wozu als Puffersubstanzen Elektrolyte eingesetzt werden müssen. Die Folge dieser Effekte ist, daß die Grenze der gebildeten Farbzone nicht scharf ausgeprägt ist und sich diese bei Durchsatz weiteren Laufmittels in Laufmittelrichtung verschiebt.

Das Prinzip der Bildung von Farbzonen, deren Länge ein Maß für die Konzentration oder Menge einer zu analysierenden Substanz in einer aufgebrachten Probenflüssigkeit darstellt, ist auch für die Analyse von Zuckern versucht worden.

Die dort vorgeschlagene Verfahrensweise führt jedoch dazu, daß das in der Reaktionszone gebildete Wasserstoffperoxid zumindest einige Minuten ohne Folgereaktion in freier Lösung verbleibt. Das in fast allen Glucoseoxydase-Präparaten enthaltene Enzym Katalase kann dann in einem unbestimmten Ausmaß Wasserstoffperoxid verbrauchen.

Das Hauptziel jener Verfahrensweise, nämlich die Bildung einer Farbzone, deren Länge ein Maß für die Menge der aufgebrachten Glucosemenge darstellt, erscheint deshalb mit den angegebenen Mitteln nicht erreichbar.

In oben erwähnter DOS 29 22 856 wird die Indikatorzone mit einer Lösung eines Chromogens, vorzugsweise o-Tolidin, getränkt. Es handelt sich hier sowohl beim Chromogen als auch bei dem gebildeten Farbstoff um Substanzen, welche eine gewisse Wasserlöslichkeit besitzen. Dadurch können sich sowohl das Chromogen, als auch der nach der Oxydation gebildete Farbstoff mit der bei der Benutzung aufsteigenden wässrigen Lösung mitbewegen. Es wurde von uns gefunden, daß ein Farbfleck von zu einem grünen Farbstoff oxydiertem Tolidin bei Konzentrationen von Tolidin und Glucose, die denen in der genannten Offenlegungsschrift vergleichbar sind, bei pH 6 und einer Wanderungsstrecke der Fließmittelfront von 32 mm je nach Wanderungsgeschwindigkeit zwischen 4 und 9 mm mit diffuser Front mitbewegt wird. Es kann sich mit der angebotenen Verfahrensweise keine exakte und reproduzierbare Grenze zwischen umgesetztem und nicht umgesetztem Teilbereich der Indikatorzoen herausbilden.

Dieser offenbar auch vom Anmelder erkannte Mangel wird dadurch abzumildern versucht, daß der Indikator in Streifen aufgetragen werden soll, zwischen denen sich gegebenenfalls farbstoffbindende Substanzen befinden können.

Das Ziel einer Ausbildung einer exakt begrenzten, gleichmäßig umgesetzten, zur Menge der aufgebrachten Substanz proportionalen Farbzone, bestehend aus zu einem eindeutigen Reaktionsprodukt umgesetzten Chromogen, wobei der gebildete Farbstoff nicht diffundieren darf, ist mit den bisher vorgeschlagenen Methoden nicht erreichbar.

Ziel der vorliegenden Erfindung ist es deshalb, die verwendeten Chromogene zu immobilisieren, ohne daß ihre Substratfunktion für das System Wasserstoffperoxid/ Peroxidase verlorengeht.

Gegenstand der Erfindung ist ein Verfahren zur Herstellung an einen Träger gebundener Verbindungen als Chromogene für in einem Träger enthaltene analytische Indikatoren, worin man eine chromogene Verbindung, gegebenenfalls über eine den Abstand zwischen chromogener Verbindung und Träger verändernde Zwischenverbindung, gegebenenfalls kovalent, an einen Träger bindet.

Gegenstand der Erfindung ist weiterhin ein diagnostisches Mittel, das aus einem streifenförmigen Träger, der die für die Testreaktion nötigen Chemikalien enthält,besteht, dadurch gekennzeichnet, daß das Chromogen- und Indikatormolekül an ein unlösliches Trägermaterial gebunden ist.

Weiterhin ist der Gegenstand der Erfindung ein Verfahren zur Herstellung eines solchen Mittels sowie die Verwendung eines an ein unlösliches Trägermaterial gebundenen Chromogens und Indikators in einem solchen Mittel.

Bevorzugt sind Mittel, bei deren Anwendung durch Reaktion einer in diesem Mittel enthaltenen Chemikalie mit einem Bestandteil der Körperflüssigkeit oder des Exkrements ein Reaktionsprodukt entsteht, das in einem als Reaktionszone bezeichneten Teil des Teststreifens eindringt und dort die Eigenschaften eines Indikators, besonders eines Chromogens, auswertbar verändert.

Es war nicht übersehbar, daß die Ausbildung von ionischen oder kovalenten Bindungen zwischen einem Träger, wie zum Beispiel Cellulose,und einem Chromogen,dessen Substratfunktion gegenüber dem System Wasserstoffperoxid/Peroxidase nicht zerstört.

Die Palette der verwendbaren Chromogene umfaßt dabei nahezu alle bisher verwendeten Chromogene, ebenso aber Substanzen, welche zwar mit dem System Wasserstoffperoxid/Peroxydase unter Farbänderung reagieren, aber aus Gründen der Stabilität oder auch wegen einer Eigenfarbe des Chromogens im reduzierten Zustand bisher keine Verwendung fanden. Daneben können auch neue, bisher nicht verwendete Chromogene verwendet werden.

Für die Herstellung der Bindung Chromogen-Träger erwiesen sich viele Möglichkeiten zur Herstellung von Bindungen prinzipiell verwendbar, so zum Beispiel Alkylierungen mittels am Träger fixierten Halogenalkyl- oder Oxirangruppen mit Chromogenen, welche Amino-, Hydroxy- oder Sulfhydryl-Gruppen enthalten. Es besteht ebenfalls die Möglichkeit, zur Herstellung von Ester- oder Thioesterbindungen zwischen Carboxylgruppen-haltigen Chromogenen und Hydroxy- beziehungsweise Sulfhydrylgruppen am Träger.

Die letzten beiden Bindungstypen wurden zum Beispiel durch Behandeln von Cellulose, welche Hydroxyalkyl- oder Sulfhydryl-Gruppen enthielt, mittels der Carbodiimid-Methode erhalten (Ch.Rev.(1967) 67, 107). Ebenso lassen sich mittels dieser Methode Amidbindungen wahlweise zwischen Carboxylgruppen-haltigen Chromogenen und Aminogruppen-haltigen Trägern wie auch umgekehrt zwischen Aminogruppen-haltigen Chromogenen und Carboxylgruppen-tragenden Trägern herstellen. Weiterhin sind mit dieser Methode Äther- und Thioäther-Bindungen mit

Hydroxyl- oder Suflhydryl-Gruppen-tragenden Chromogenen herstellbar.

Ferner wurden mittels Kupplungsreaktionen polymer gebundene Chromogene erhalten. Es erwies sich dabei allgemein als vorteilhaft, das zu bindende Chromogen in genügend großem Abstand von der Matrix des Polymeren zu fixieren, wie beispielsweise in dem trägergebundenen Chromogen der Formel I.

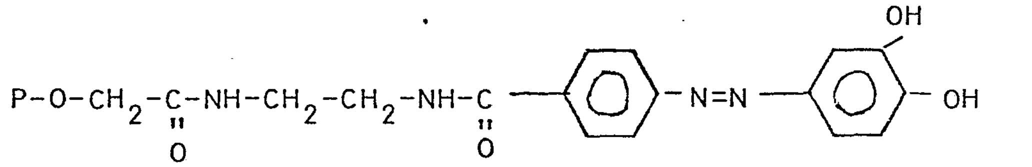 I

P = Polymer.

Verbindung I ist ein gelbes Pulver, welches verglichen mit reinem Brenzkatechin eine größere Stabilität gegen Oxydation an der Luft oder in wässriger Lösung aufweist.

Als Polymere kommen besonders solche in Betracht, die Hydroxigruppen tragen, insbesondere Cellulose.

Ganz allgemein wurde beobachtet, daß die Oxydationsempfindlichkeit durch Herstellung einer Bindung an einen Träger verbessert wird. Zum Beispiel zeigt o-Dianisidin, das mittels Bromacetyl-Gruppen an Cellulose gebunden wurde, nach mehr als drei Jahren an der Luft keine wahrnehmbare Eigenoxydation. Bevorzugte Verwendung fanden als Chromogene Vertreter der Benzidinreihe, welche über langkettige Spacer an den Träger, bevorzugt Cellulose, gekoppelt wurden. Diese Substanzen haben große Stabilität gegenüber Oxydation an der Luft. Sie sind mit vergleichsweise hoher Geschwindigkeit direkt enzymatisch oxydierbar.

Ausreichende Stabilität in feuchtem Zustand ist dagegen an die Einhaltung bestimmter Bedingungen geknüpft. Mit destilliertem Wasser benetzt waren die meisten verwendeten polymer gebundenen Chromogene über mehrere Tage völlig stabil. Verschlechtert wird die Stabilität durch das Vorhandensein gewisser Ionen, wobei viele zweiwertige Kationen, insbesondere aber Fe(III)-Ionen die Oxydation feuchter Chromogene katalysieren. In diese Richtung wirken auch einige, vorwiegend mehrwertige Anionen.

Diese Eigenschaften haben Rückwirkung auf die Wahl geeigneter Puffersysteme. Beispielsweise wird das in Ausführungsbeispiel 1 beschriebene o-Dianisidin-Präparat bei pH-Werten um 4 in einem Acetat-Puffer kaum, dagegen in einem Citrat-Puffer stärker autokatalytisch oxydiert.

Es ergeben sich dabei erhebliche Unterschiede innerhalb der vorzugsweise verwendeten Substanzklasse.

Die Oxydationsempfindlichkeit des polymer gebundenen Tolidins ist dabei besonders groß. Bei pH-Werten von 4 - 5 wird über einen Spacer gebundenes Tolidin in Gegenwart von Peroxidase in wenigen Minuten sichtbar gebläut, was möglicherweise auch durch aus dem Enzympräparat in Spuren austretenden Eisenionen begünstigt wird. Besser ist die Stabilität des polymer gebundenen o-Dianisidins.

Die Stabilität kann dabei durch Folgeumsetzungen der polymer gebundenen Chromogene weiter verbessert werden.

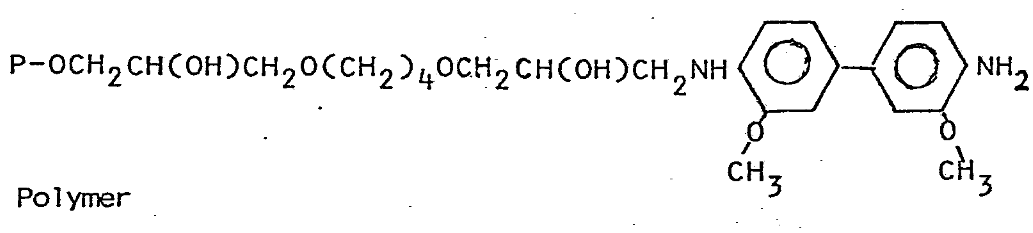

P = Polymer

II

Während das polymer gebundene o-Dianisidin(Formelbild II) bereits ausreichende Stabilität aufweist, wird durch Folgeumsetzung mit stöchiometrischen Mengen 2-Chloräthanol und Tri-n-butylamin in Dimethylformamid ein Präparat erhalten, welches bei Verwendung in Teststäbchen keine erkennbare Eigenoxidation oberhalb der gebildeten Farbzone aufweist. Bei der vorzugsweise verwendeten Substanzklasse handelt es sich um mehrstufige Red-Ox-Systeme. Wegen der für das vorliegende Problem notwendigen Forderung nach Stöchiometrie ist die Bildung eines definierten Oxidationsproduktes wünschenswert.

Die über einen Spacer polymer gebundenen Chromogene aus der Klasse der Benzidine reagieren bei niedrigen pH-Werten größtenteils zu intensiv farbigen Zwischenstufen im Farbbereich grün-blau. Bei pH-Werten um 7 wird daneben die nächsthöhere, zum Beispiel beim o-Dianisidin bei allen durchgeführten Bindungstypen im Bereich brauner Farbtöne liegende, Oxydationsstufe gebildet.

Die pH-abhängige Ausbildung der einzelnen Oxydationsstufen hat dabei Einfluß auf die Länge des gebildeten Farbstreifens.

Weiterhin ist die Geschwindigkeit der Reaktion mit Wasserstoffperoxid/Peroxidase ein wichtiger Parameter. Das in vielen handelsüblichen Peroxidase-Präparaten enthaltene Enzym Katalase verbraucht Wasserstoffsuperoxid, weshalb die Menge an Peroxidase möglichst klein gehalten werden sollte.

Dies kann dadurch umgangen werden, daß mittels einer intermediären und leicht durch das System Wasserstoffperoxid/Peroxydase oxydierbaren Re-Ox-Systems das gebildete Wasserstoffperoxid verbraucht wird umd das intermediär gebildete Oxydationsmittel mit einem polymer gebundenem Chromogen reagiert.

Als intermediäres Oxydationsmittel kann ein wasserlösliches Jodid vorteilhaft verwendet werden.

Das mittels Wasserstoffperoxid/Peroxydase aus Jodid gebildete Jod ist in der Lage, eine Reihe von trägergebundenen Farbstoffen zu entfärben, beispielsweise Neutralblau, Gallocyanin oder Indigocarmin, oder auch trägergebundene Leukofarbstoffe zu den entsprechenden Farbstoffen zu oxydieren.

Auch viele der direkt oxydierbaren polymer gebundenen Chromogene kommen für diese Reaktion in Frage.

Beispielsweise reagiert Jod mit über einem Spacer an Cellulose gebundenem o-Dianisidin, wie auch o-Tolidin und Benzidin, unter Bildung von grünlich-blauen Farbstoffen.

Zur Verhinderung einer Oxydation des Trägers mit Jod muß gegebenenfalls Jodid als Komplexbildner zugegeben werden. Im allgemeinen genügen etwa 10 Gew.% gemessen an der Menge des polymer gebundenen Chromogens.

Für die Präparation der Reaktionszone der Teststäbchen ist es wesentlich, daß die Enzyme Peroxydase und Glucoseoxydase immobilisiert werden. Dies ist bei pH-Werten unter 6 in genügendem Ausmaß bereits mit einem genügend stark sauren Kationenaustauscher, welcher vorher in die $Na^+$- oder $K^+$-Form gebracht wurde, der Fall. Peroxidase wird stark, Glucoseoxydase mäßig gebunden. Bezüglich Glucoseoxydase zeigt quarternierte Diäthylaminocellulose ebenfalls Immobilisierungswirkung.

Im allgemeinen ist ein Zusatz von Mutarotase nicht erforderlich. Das bevorzugt verwendete Glucoseperoxydase-Präparat zeigte, obwohl hochgereinigt, ausreichende Mutarotaseaktivität, so daß selbst bei einer Analysendauer

von weniger als 5 Minuten eine gleichmäßig farbige und scharf abgegrenzte Farbzone umgesetzten Chromogens erhalten wurde.

Die zu analysierende Flüssigkeit, beispielsweise Blut oder Harn, kann mittels eines saugfähigen Trägers zugeführt werden, wobei durch zeitlich terminiertes Benetzen einer definierten Zone eine definierte Substanzmenge aufgebracht werden kann. Besser geeignet als beispielsweise Papier, dessen Saugfähigkeit von der Luftfeuchtigkeit abhängt, sind Membranfilter. Als Basismaterial hierfür eignen sich beispielsweise die in bekannten Verfahrensweisen herstellbaren Celluloseester, insbesondere Cellulose-2,5-Nitrat oder Cellulose-Acetat. Falls ionisch gebundene Chromogene verwendet werden, muß ggfs.entsalzt werden. In einer bevorzugten Ausführungsform werden in bekannter Weise hergestellte Lösungen dieser Materialien in die Aussparung des in den Figuren 1 und 2 gezeigten Stäbchens gegossen und überschüssiges Material entfernt. Das Stäbchen besteht vorzugsweise aus Kunststoff und ist mit einer sich längs erstreckenden Aussparung versehen, die sich verjüngen kann. Durch Verdunsten des Lösungsmittels bildet sich dann das Filtermaterial auf dem Boden. Dabei kann auf der Rückseite des Stäbchens eine Teilfläche der Membrane unbedeckt bleiben, das heißt, das Stäbchen weist eine Öffnung auf, die während des Beschichtens mit einem Material verschlossen ist, auf welchem das Filtermaterial eine geringe Haftung aufweist, wie zum Beispiel Polyäthylen oder Polystyrol.

Wegen der guten Haftung der Membranfilter auf Plexiglas diente dieses Material vorzugsweise als Grundkörper bei der Herstellung von Teststäbchen.

Die Verwendung einer Membranfilterschicht als Aufbringzone hat folgende weiteren Vorteile: Bei Porenweiten im Bereich von 0,6 bis 1,5 Mikron hat das Material Filtereigenschaften für alle Blutzellen und die Katalase wird immobilisiert. Dieses Enzym befindet sich in den Blutzellen und wird zum Beispiel bei der kaum völlig auszuschliessenden Hämolyse beim Aufbringen von Vollblut freigesetzt.

Zur Entfernung von störenden Bestandteilen, insbesondere bei einer Harnzuckeranalyse, kann eine Zone aus Kat- und Anionenaustauschern (Mischbett) im Anschluß an die Aufbringzone angeordnet werden. Diese kann aus Cellulose-Ionenaustauschern oder auch aus Anionen- und Kationenaustauschern auf der Basis quervernetzter Styrol-Divinylbenzol-Produkte hergestellt werden. Das Gemisch der Ionenaustauscher wird mit einer Pufferlösung geeigneten pH-Wertes vorbehandelt und dann mit oder ohne Bindemittel als separate Zone unterhalb der Reaktionszone aufgebracht. Die Ionenstärke des Puffers, welcher in die Reaktionszone gelangt, wird dabei von der Ionenstärke des Fließmittels bestimmt.

Bei einer Blutzuckeranalyse kann dabei ein pH-Gradient im Bereich der Ionenaustauschzone eingestellt werden, wenn mit sauren oder alkalischen Lösungen gearbeitet wird, um störende Proteine zu fällen und zu immobilisieren.

Zur Reduzierung der Fließgeschwindigkeit auf Werte, unter denen ein vollständiger Umsatz der polymer gebundenen Chromogene im Tempo der Benetzung mit Fließmittel ermöglicht wird, kann an einer beliebigen Stelle unterhalb der Reaktionszone eine Zone Membranfiltermaterial entsprechender Porenweite aufgebracht werden. Analog zur Glucose-Bestimmung kann das beschriebene Testmaterial bei anderen Substanzen angewendet werden, welche Substrate von Enzymen sind, die bei der Oxydation der Substrate Wasserstoffperoxyd erzeugen.

Weitere Anwendungsmöglichkeiten trägergebundener Chromogene und Indikatoren sind demnach zum Beispiel der quantitative Nachweis von Cholesterin mittels Cholesterinoxydase oder der entsprechende Nachweis von Galaktose mittels Galaktose-Oxydase.

Im folgenden wird die Erfindung an Beispielen erläutert.

Beispiel 1

Herstellung des trägergebundenen Chromogens

0,5 g Cellulosepulver, durchschnittliche Partikelgröße 10 Mikron, werden in einer Mischung von 3 ml 0,2 M Natronlauge und 1,5 ml Butandiol-1,3-diglycidyläther während 20 Stunden bei 25°C gerührt. Danach wird abgesaugt, mit 0,2 M Kochsalzlösung gewaschen, bis ein neutrales Filtrat erhalten wird. Es werden 0,5 g o-Dianisidin in 10 ml Dimethylformamid gelöst und mit 10 ml einer entgasten 0,2 M Natriumhydrogencarbonat-Lösung unter Stickstoffdurchleitung vermischt. Mit 2 M Natronlauge wird ein pH von 10 eingestellt und 0,5 g Polyäthylenglykol 4000 zugegeben. Das Cellulose-Präparat wird zugegeben und die Mischung 2 Stunden unter Stickstoffdurchleitung bei 80°C gerührt. Es wird abgesaugt, mehrmals mit Dimethylformamid gewaschen, bis das Filtrat frei von o-Dianisidin ist, und mehrmals mit 0,2 M Kochsalzlösung, 0,5 M Essigsäure, zweimal mit destilliertem Wasser und zweimal mit Methanol gewaschen und an der Luft getrocknet.

Dieses Produkt ist mittels eines Spacers an Cellulose gebundenes o-Dianisidin. Das Produkt ist Substrat des Systems Wasserstoffperoxyd/Peroxydase und wird von diesem

im pH-Bereich 3,5 - 6 zu einem intensiv grünen Farbstoff oxidiert. 0,2 g dieses Produktes werden in 5 ml Dimethylformamid suspendiert, 0,0014 ml 2-Chloräthanol und 0,0095 ml Tri-n-butylamin zugegeben und unter Rühren 4 Stunden bei 100°C erhitzt, das Produkt abgesaugt, mit Dimethylformamid, mehrmal mit 0,2 M Kochsalzlösung, mit destilliertem Wasser und zweimal mit Methanol gewaschen und an der Luft getrocknet. Das erhaltene Produkt weist alle Vorzüge des Vorproduktes bezüglich der enzymatischen Oxydierbarkeit auf, besitzt aber eine größere Stabilität gegenüber autokatalytischer Oxydation an der Luft in feuchtem Zustand.

<u>Beschichtung der Teststäbchen</u> (1 in Fig. 1 und 2)

Zunächst werden die Lösungen zur Beschichtung der einzelnen Zonen hergestellt.

Reaktionszone (2) mit Auffangzone(7): 0,4 g trägergebundenes Chromogen werden mit 0,1 g Sulfoäthylcellulose, Natrium-Form, 0,1 g quarternierter Diäthylaminocellulose, Chlorid-Form, und 3 ml 0.1 M Dimethylglutarsäure-Puffer pH 4,0 gemischt.

Ionenaustauschzone (3) : 0,05 g quartäre Ammoniumgruppen enthaltenes Anionen-Austauscher-Harz (OH-Form) sowie 0,05 g Sulfonsäure-Gruppen enthaltenes Kationen-Austauscher-Harz, $H^+$-Form, (beide Formen gemahlen) werden mit 0,12 ml 90 % n-Propanol getränkt und mit 0,3 ml einer unten (s.Aufbringzone) beschriebenen Membranfilter-Lösung mit allerdings höherem Wassergehalt vermischt.

Aufbringzone (4): 5 g Cellulose-2,5-Nitrat werden in 55 g Essigsäuremethylester gelöst. Dann werden zugegeben: 24 g absoluter Alkohol, 12,3 n-Butanol, 1,5 g Glycerin und 3,2 g Wasser.

Zone zur Regulierung der Fließgeschwindigkeit(5): Hierzu kann eine Lösung entsprechend der für die Aufbringzone dienen. Der Wassergehalt der Lösung bestimmt dabei die Porenweite des nach dem Trocknen entstehenden Filters und kann hier höher liegen als in der Aufbringzone.

Je etwa 30 Stück Stäbchen werden auf eine Unterlage gelegt, die Passungen für die Öffnungen (6) in den Stäbchen besitzt, und zunächst die für die Beschichtung mit Membranfiltermaterial vorgesehenen Zonen abgedeckt.

Es wird unter ständigem Rühren der Imprägnierungslösungen die Reaktionszone (2) beschichtet.

Nach leichtem Antrocknen werden die Abdeckungen entfernt und die restlichen Zonen werden mit den Membranfilterlösungen beschichtet. Diese Lösungen gelieren sofort, wenn sie mit noch feuchten Grenzschichten in Berührung kommen. Nach dem Trocknen unter definierten Bedingungen, beispielsweise 70% Luftfeuchtigkeit und 25°C, werden die Stäbchen mit Enzymlösung beschichtet. Dies kann automatisch mittels einer Vorrichtung geschehen, wobei mittels

eines Dochtes jeweils eine definierte Enzymmenge aufgebracht wird. Es genügen je 2 - 5 Mikrogramm Glucoseoxydase und Peroxydase (Aktivität je etwa 250 U).

Zuletzt werden die Stäbchen vorsichtig von der Unterlage gelöst und es kann ein Papierstreifen mit Graduierung angeklebt werden.

Die Membranfilterlösung hat sich zu einem am Boden haftenden Filter verfestigt. Bei einer Aufbringungsdicke von 1 mm und einem Wassergehalt von 3,2% bildet sich ein 0,06 mm dickes Filtermaterial mit etwa 0,6 Mikron Porenweite. Ein derartiges Filtermaterial ist beispielsweise zur Prüfung von Harn geeignet. Für die Prüfung von Vollblut wird die Porenweite zweckmäßigerweise durch einen entsprechend höheren Wassergehalt der Lösung für die Aufbringzone auf Werte zwischen 1 und 2 Mikron erhöht.

Das mit Analysenlösung beschickte Teststäbchen kann nun mittels der in Fig.3 skizzierten Vorrichtung in waagerechter oder leicht geneigter Lage für die Analyse eingesetzt werden. Natürlich kann dies auch durch Hineinstellen in ein entsprechendes Gefäß geschehen, auf dessen Boden sich das Fließmittel befindet.

Das Teststäbchen wird in eine Passung (8) der in Fig.3 dargestellten Vorrichtung gelegt. Die an einem Ende mit zwei Halterungen versehene S-förmige Feder 9 dient nach Einschieben eines Papierstreifens als Flüssigkeitstransportbrücke zwischen dem Fließmittelreservoir 10 und dem unteren Ende des Teststäbchens. Die mit einem Papierstreifen beschickte Feder wird in die Einschnitte 11 geschoben. Dann wird eine aus durchsichtigem Material bestehende Abdeckung 12 in die Führungsrillen 13 geschoben. Dabei wird vom Deckel der Abdeckung 12 die Feder nach unten gedrückt und die Flüssigleitsbrücke geschlossen. Gleichzeitig wird das Gefäß abgeschlossen und der Fortgang der Analyse kann vom Anwender beobachtet werden.

Das die Zonen nacheinander durchströmende Fließmittel erzeugt einen zur Menge der aufgebrachten Glucose proportional langen Farbstreifen.

Beispiel 2

Herstellung trägergebundenen p-Phenylendiamins als Chromogen

10 g Cellulose-Pulver, durchschnittliche Partikelgröße 10 Mikron, werden über Nacht in 100 ml 10%iger methanolischer Kalilauge suspendiert. Die überschüssige Lauge wird abgepresst, die erhaltene Alkalicellulose in eine Lösung von 8 g Chloressigsäureamid in 120 ml absolutem Alkohol eingetragen und bei 50°C eine Stunde gerührt.

Dann wird abgesaugt, das Cellulose-Produkt mehrmals mit 0,2 M Kochsalzlösung bis zur neutralen Reaktion des Filtrates gewaschen, mehrmals mit destilliertem Wasser und danach mit Methanol gewaschen und an der Luft getrocknet.

Das so erhaltene Cellulose-Carboxamid-Präparat wird in 100 ml Hexamethylendiamin bei 90°C eingetragen und 8 Stunden bei dieser Temperatur gerührt. Die Suspension wird heiß gefiltert, mehrmals mit Methanol und so lange mit 0,2 M Kochsalzlösung gewaschen, bis ein neutrales Filtrat erhalten wird. Es wird mehrmals mit destilliertem Wasser und dann mit Methanol gewaschen,

und das Material an der Luft getrocknet.

Das erhaltene Produkt enthält über Carboxamid Gruppen gebundene Hexylamino-Gruppen mit einer Kapazität von etwa 0,1 Mol/kg.

10 g dieses Produkts werden in 150 ml 0,2 M Kochsalzlösung suspendiert und eine Lösung von 0,4 g p-Nitrobenzoylazid (Org. Syntheses 4, 716) in 150 ml Dimethylformamid zugegeben. Unter Rühren wird 0,17 ml Triäthylamin zugegeben und eine Stunde gerührt. Das Produkt wird abgesaugt, mit Dimethylformamid, 0,2 M Kochsalzlösung, destilliertem Wasser und Methanol gewaschen und an der Luft getrocknet. Das Produkt enthält über Carboxamid-Gruppen gebundenes p-Nitrobenzoesäurehexylamid.

Dieses Produkt wird in 150 ml einer 0,5 M Natriumhydrogencarbonat-Lösung eingetragen. Nach Erwärmen auf 40°C werden portionenweise 5 g Natriumdithionit unter Rühren zugegeben und noch 30 Minuten bei dieser Temperatur gerührt.

Das Produkt abgesaugt, mehrmals mit 0,2 M Kochsalzlösung, destilliertem Wasser und Methanol gewaschen und an der Luft getrocknet. Es enthält über Carboxamid-Gruppen gebundenes p-Aminobenzoesäurehexylamid.

1 g dieses Produktes wird in 20 ml einer eiskalten 0,5 M Salzsäurelösung eingetragen, und es werden portionenweise kleine Mengen Natriumnitrit in Abständen von 3-5 Minuten zugegeben, bis ein jeweils mit Kaliumjodid-Stärkepapier durchgeführter Test überschüssiges Nitrit anzeigt. Danach wird noch 10 Minuten gerührt und in kleinen Portionen Amidoschwefelsäure zugegeben, bis alles

überschüssige Nitrit zerstört ist.

Nun wird schnell abgesaugt und das diazotierte Produkt in eine Lösung von 2 g p-Phenylendiamin in einem Gemisch von 50 ml Dimethylformamid und 50 ml Acetatpuffer, welcher auf einen pH-Wert von 4,2 eingestellt war, bei einer Temperatur von 0°C eingetragen und eine Stunde bei dieser Temperatur gerührt.

Dann wird abgesaugt, mehrmals mit Dimethylformamid, verdünnter Essigsäurelösung und 0,2 M Kochsalzlösung gewaschen, bis ein neutrales Filtrat erhalten wird. Dann wird mit destilliertem Wasser und Methanol gewaschen, und das Produkt an der Luft getrocknet.

Das entstandene leicht rosa-violette Pulver ist das Kupplungsprodukt des p-Phenylendiamins mit dem diazotierten Arylamino-Derivat der Cellulose.

Dieses polymer gebundene Derivat des p-Phenylendiamins ist ein Substrat für das System Wasserstoffperoxyd-Peroxydase und wird von diesem zu einem braunen Farbstoff oxydiert.

Beispiel 3

Herstellung trägergebundenen o-Dianisidins als Chromogen

5 g Cellulosepulver werden 1 Tag bei Zimmertemperatur mit 12%iger methanolischer Kalilauge behandelt. Das Produkt wird von der Lauge abgepreßt, portionenweise in eine Lösung von 8 g Chloressigsäureamid in 100 ml absolutem Alkohol eingetragen und 30 Minuten bei 45°C gerührt.

Dann wird abgepreßt, mehrmals mit absolutem Alkohol, 0,2 M Kochsalzlösung, destilliertem Wasser und zweimal mit Methanol gewaschen und das Produkt an der Luft getrocknet. Das erhaltene Cellulose-Carboxamid Präparat wird in 50 ml Äthylendiamin eingetragen und 5 Stunden bei 90°C gerührt. Danach wird abgesaugt, mehrmals mit Methanol und so lange mit 0,2 M Kochsalzlösung gewaschen, bis ein neutrales Filtrat erhalten wird. Danach wird mehrmals mit destilliertem Wasser und zweimal mit Methanol gewaschen und das Produkt an der Luft getrocknet.

Das Produkt enthält über Carboxamid Gruppen gebundene Äthylamino-Gruppen.

Nun werden 1 Millimol Bromessigsäure (139 mg) und 1,1 Millimol N-Hydroxysuccinimid (127 mg) in 8 ml Dioxan gelöst und 1,2 Millimol (248 mg) Dicyclohexylcarbodiimid (DCC) in fester Form zugegeben und gelöst. Nach einer Stunde wird vom gebildeten Dicyclohexylharnstoff abgefiltert und das Filtrat, welches den aus Bromessigsäure- und N-Hydroxysuccinimid gebilteten Ester enthält, in 42 ml Phosphatpuffer von ph 7,5 bei einer Temperatur um 0°C eingerührt. 1 g der Äthylaminogruppen-haltigen Cellulose wird zugegeben und die Mischung 30 Minuten bei Temperaturen um 0°C gerührt. Danach wird abgesaugt, zweimal mit Dioxan/Wasser Gemisch (50/50 v:v) und danach mehrmals mit eiskalter 0,2 M Kochsalzlösung gewaschen.

Das Produkt enthält über Amidbindungen an Cellulose gebundene Bromacetyl-Gruppen.

Das Produkt wird sofort unter Durchleiten von in Stickstoff eine Lösung von 3 g o-Dianisidin in 25 ml Dimethylformamid und 25 ml 0,2 M Natriumhydrogencarbonat-Lösung,

welche mit 2 N Salzsäure auf einen pH-Wert von 7,8 eingestellt war, eingetragen und 2 Stunden bei 55°C erwärmt. Danach wird abgesaugt, mehrmals mit Dimethylformamid, 0,2 M Kochsalzlösung, destilliertem Wasser und zweimal mit Methanol gewaschen und das Produkt an der Luft getrocknet. Das erhaltene Produkt ist ein Substrat für das System Wasserstoffperoxyd/Peroxydase und wird von diesem bei pH-Werten um 7 zu einem braunen Farbstoff oxydiert.

Beispiel 4

Herstellung eines ionogenen gebundenen Chromogens

3 g Cellulosepulver werden in einer Mischung von 6 ml 0,6 N Natronlauge, 2 ml Butandiol-1,3-diglycidäther und 10 ml Wasser über Nacht bei 25°C gerührt. Es wird abgesaugt, mit 0,1 M Kochsalzlösung bis zum Erhalt eines neutralen Filtrates und mehrmals mit destilliertem Wasser und zweimal mit Methanol gewaschen und an der Luft getrocknet. Das etwa 0,06 mol/kg Oxiran-Gruppen enthaltende Produkt wird in einer Mischung von 3 ml N,N-Diethyl-ethylendiamin, 6 ml destilliertem Wasser und 9 ml Dimethylformamid während 2 Stunden bei 70°C gerührt. Dann wird abgesaugt, mit destilliertem Wasser bis zum Erhalt eines neutralen Filtrates und zweimal mit Methanol gewaschen und an der Luft getrocknet.

Das erhaltene Produkt enthält etwa 0,05 mol/kg über einen Spacer gebundene Diethylaminogruppen.

1,5 g dieses Produktes werden in einer Mischung von 3 ml Methyljodid und 10 ml absolutem Alkohol über Nacht bei 40°C gerührt. Danach wird abgesaugt, mehrmals mit absolutem Alkohol, mit destilliertem Wasser und mehrmals mit Methanol gewaschen und an der Luft getrocknet.

Das Produkt enthält etwa 0,05 mol/kg quartäre Ammoniumgruppen.

Benzidin-3,3-dicarbonsäure wird gemäß Fierz-David und Blangey, Farbenchemie, Springer Verlag 1946, S.157-159 hergestellt.

Benzidin-3,3-dicarbonsäure ist ein Substrat des Systems Wasserstoffperoxid-Peroxidase und wird von diesem bei pH-Werten um 7 zu einen intensiv blauen Farbstoff oxydiert.

1 mmol (0,345 g) Benzidin-3,3-dicarbonsäure-dihydrochlorid werden in einer Mischung von 2 mmol 2-Chlorethanol (0,135 ml) und 6 mmol 1,2,2,6,6-Pentamethyl-piperidin (1,08 ml) in 20 ml Dimethylformamid während 1 Stunde bei 100°C gerührt. Nach Abkühlen wird gefiltert und die Hälfte des Filtrates mit 7 ml destilliertem Wasser verdünnt und während 10 min. mit 0,2 g des oben beschriebenen Cellulose-Ionenaustauschers gerührt. Dann wird abgesaugt, mit Dimethylformamid und Dimethylformamid-Wasser 50:50 (v/v) gewaschen und der Feststoff unter denselben Bedingungen 10 min. mit dem Rest des Filtrates gerührt. Nach Absaugen wird mit Dimethylformamid, Dimethylformamid-Wasser 50:50 (vv), destilliertem Wasser und mehrmals mit Methanol gewaschen, und das Produkt an der Luft getrocknet.
Das erhaltene leicht violette Pulver ist Substrat des Systems Wasserstoffperoxid-Peroxidase und wird in entionisierter Lösung zu einem intensiv grün-schwarzen Farbstoff oxydiert.

Mit Hilfe dieses Produktes läßt sich ebenfalls ein Teststäbchen zum quantitativen Nachweis von Wasserstoffperoxid oder einer Substanz eines unter Bildung von Wasserstoffperoxid reagierenden Enzym-Substrat-Systems herstellen.

Dazu kann die in dem Ausführungsbeispiel 1 beschriebene Ionenaustauschzone mit Kationenaustauschern der $H^+$-Form und Anionenaustauschern der OH-Form beschichtet werden. Die Reaktions- und Nachweiszone kann neben dem polymeren Chromogen Kationenaustauscher der $H^+$-Form (zum Beispiel Carboxymethylcellulose) und Anionenaustauschern der OH- oder Hydrogencarbonat-Form (beispielsweise fein gemahlenes

Anionenaustauscher-Harz auf der Basis Styrol-Divinylbenzol) enthalten.
Hierdurch werden in einem enzymkatalysierten Prozess entstehende Elektrolyte gebunden, und es ergibt sich ein entionisiertes Medium.

Das Chromogen besitzt eine große Affinität zu dem verwendeten Cellulose-Ionenaustauscher und wird durch Elektrolyte nur in geringem Umfang mobilisiert.

Die Reaktions- und Nachweiszone kann daher auch mit Puffersystemen geringerer Ionenstärke versetzt werden.
Dazu eignen sich beispielsweise bipolare Puffersubstanzen geeigneten pH-Wertes, wie N-Morpholino-3-propansulfonsäure.

Patentansprüche :

1. Verfahren zur Herstellung an einen Träger gebundener Verbindungen als Chromogene für in einem Träger enthaltene analytische Indikatoren, dadurch gekennzeichnet, daß man die chromogene Verbindung, gegebenenfalls über eine den Abstand zwischen Träger und chromogener Verbindung verändernde Zwischenverbindung, an den Träger bindet.

2. Verfahren nach Anspruch 1, dadurch gekennzeichnet, daß der Träger eine Polyhydroxiverbindung ist.

3. Verfahren nach Anspruch 1, dadurch gekennzeichnet, daß die chromogene Verbindung kovalent an den Träger gebunden wird.

4. Verbindung der Formel

Polymer-$OCH_2CH(OH)CH_2O(CH_2)_4OCH_2CH(OH)CH_2NH$ 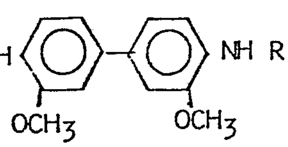

worin R = H,$-CH_2CH_2OH$ ist.

5. Eine Verbindung der Formel

Polymer-$OCH_2CONH(CH_2)_6NHCO$ 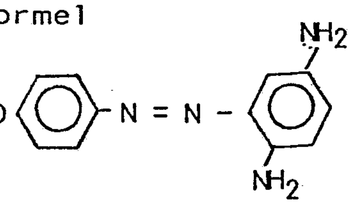

6. Eine Verbindung der Formel

Polymer-$OCH_2CONHCH_2CH_2NHCOCH_2NH$ 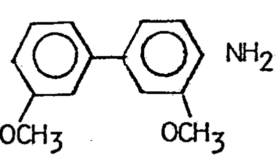

7. Diagnostisches Mittel enthaltend ein Chromogen nach einem der Ansprüche 1 bis 6.

8. Verwendung eines Chromogens nach einem der Ansprüche 1 bis 6 zum Nachweis und zur Bestimmung eines Bestandteils einer Körperflüssigkeit oder eines Exkrements.

9. Diagnostisches Mittel zum Nachweis und zur Bestimmung von Glucose und einer Flüssigkeit, enthaltend ein Chromogen nach einem der Ansprüche 1 bis 6 als Indikator in einer Vorrichtung zusammen mit einem zur Aufnahme der zu analysierenden Flüssigkeit dienenden saugfähigen Material und einem damit in Kontakt stehenden Mischbett-Ionenaustauscher sowie gegebenenfalls einem Bindemittel, gegebenenfalls verbunden über eine Zone definierter Saugfähigkeit.

FIG.1

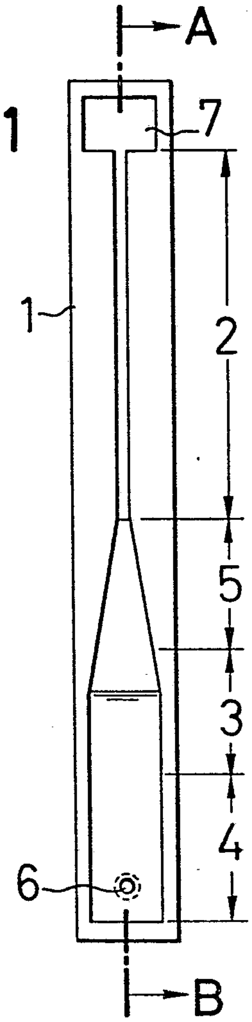


FIG. 2

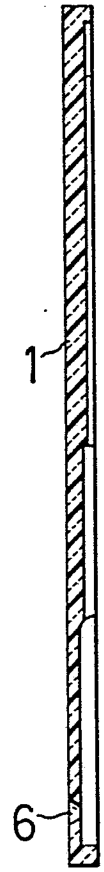


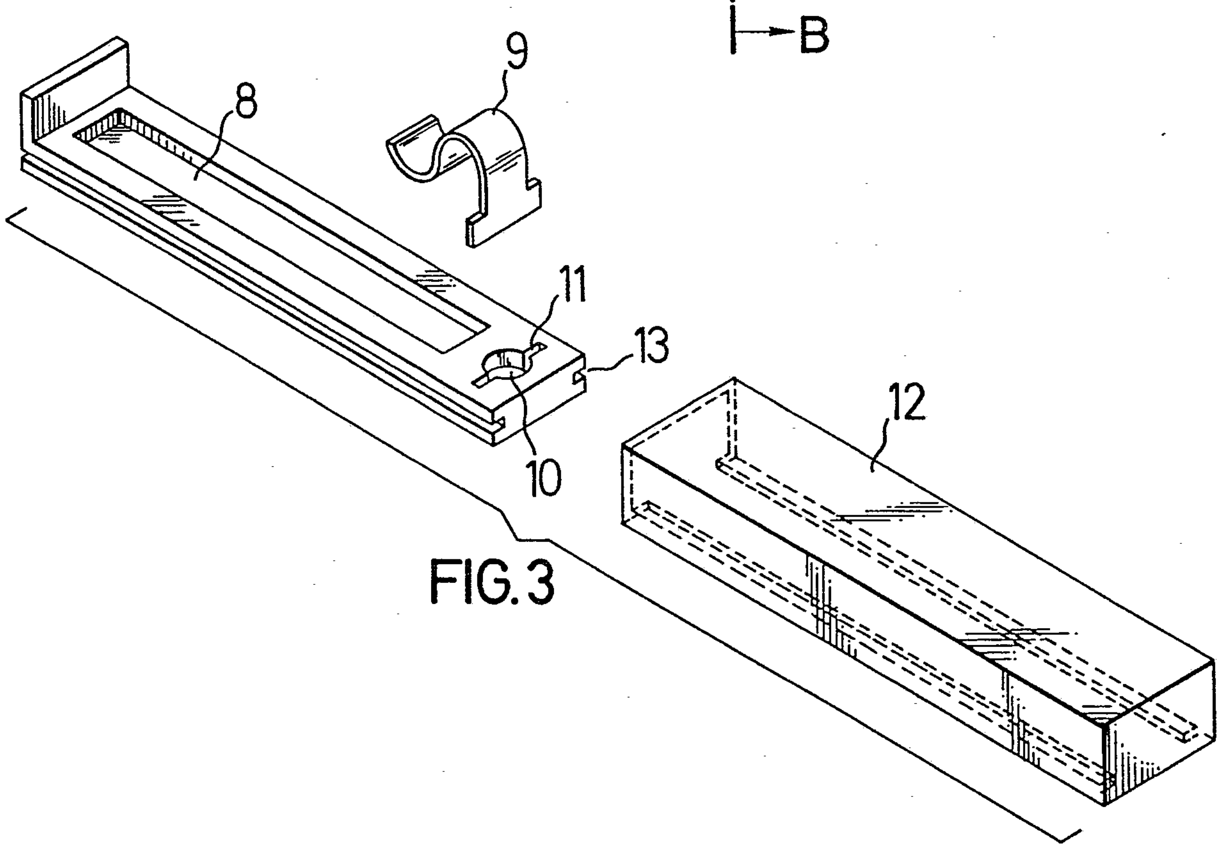


FIG.3

Europäisches Patentamt

# EUROPÄISCHER RECHERCHENBERICHT

0039027
Nummer der Anmeldung

EP 81 10 2985

| Kategorie | EINSCHLÄGIGE DOKUMENTE<br>Kennzeichnung des Dokuments mit Angabe, soweit erforderlich, der maßgeblichen Teile | betrifft Anspruch | KLASSIFIKATION DER ANMELDUNG (Int. Cl.³) |
|---|---|---|---|
| | J. OF ANALYTICAL CHEMISTRY USSR, Band 32, Nr. 911, September 1977, Seiten 1442-1454<br>V.M. OSTROVSKAYA: "Chromogenic analytical reagents fixed to supports"<br>* Insgesamt *<br>-- | 1-3,7-9 | G 01 N 33/52<br>C 12 Q 1/00<br>G 01 N 31/22 |
| | BE - A - 698 813 (DAINICHISEIKA COLOR & CHEMICALS MFG. CO. LTD.)<br>* Seite 2, Zeile 11 - Seite 4, Zeile 9; Seiten 17-19; Ansprüche 1-3,6,8 *<br>-- | 1-3 | |
| | DE - A - 1 956 214 (MILES LABORATORIES INC.)<br>* Ansprüche 6,7 *<br>-- | 1,3,7,8 | RECHERCHIERTE SACHGEBIETE (Int. Cl.³)<br>C 12 Q 1/00<br>1/28<br>1/54<br>G 01 N 31/22<br>33/52<br>33/62<br>33/64<br>33/66<br>33/72<br>C 09 B 69/00 |
| A | DE - A - 2 247 163 (MERCK PATENT GmbH)<br>* Insgesamt *<br>-- | 4-6 | |
| A | DE - A - 1 745 121 (MILES LABORATORIES INC.) | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| A | MACROMOLECULES, Band 11, Nr. 2, März-April 1978, Seiten 357-359<br>H.R. ALLCOCK et al.: "Poly(organophosphazenes) with chromophores as substituent groups"<br>-- | | |
| A | US - A - 3 904 373 (G.B. HARPER)<br>---- | | |

X Der vorliegende Recherchenbericht wurde für alle Patentansprüche erstellt.

| Recherchenort | Abschlußdatum der Recherche | Prüfer |
|---|---|---|
| Den Haag | 04-08-1981 | GRIFFITH |

EPA form 1503.1 06.78